# EUROPEAN PATENT SPECIFICATION

(11) **EP 1 534 139 B1**
(45) Date of publication and mention of the grant of the patent: **19.12.2018**
(21) Application number: 03793365.2
(22) Date of filing: 25.08.2003
(51) Int. Cl.: A61B 8/12, A61B 8/00, A61B 5/02, A61B 5/0456, G06T 7/00, G06T 7/12, A61B 8/08

(54) **SYSTEM AND METHOD OF CHARACTERIZING VASCULAR TISSUE**
SYSTEM UND VERFAHREN ZUR CHARAKTERISIERUNG VON GEFÄSSGEWEBE
SYSTEME ET PROCEDE PERMETTANT DE CARACTERISER UN TISSU VASCULAIRE

(30) Priority: 26.08.2002 US 406183 P; 26.08.2002 US 406254 P; 26.08.2002 US 406184 P; 26.08.2002 US 406185 P; 26.08.2002 US 406234 P
(43) Date of publication of application: 01.06.2005
(73) Proprietor: THE CLEVELAND CLINIC FOUNDATION, Cleveland, OH 44195 (US)
(72) Inventor: NAIR, Anuja, Copley, Ochio 44321 (US); VINCE, Geoffrey, D., Avon Lake, OH 44012 (US); KLINGENSMITH, Jon, D., Shaker Heights, OH 44120 (US); KUBAN, Barry, D., Avon Lake, OH 44012 (US)
(74) Representative: de Haan, Poul Erik
(86) International application number: PCT/US2003/026521
(87) International publication number: WO 2004/017835

(56) References cited:
- US-A- 5 876 343
- US-A- 6 120 445
- US-B1- 6 200 268
- US-B1- 6 200 268
- US-B2- 6 514 203
- NAIR A ET AL: "Assessing spectral algorithms to predict atherosclerotic plaque composition with normalized and raw intravascular ultrasound data" ULTRASOUND IN MEDICINE AND BIOLOGY, NEW YORK, NY, US, vol. 27, no. 10, 1 October 2001 (2001-10-01), pages 1319-1331, XP004324335 ISSN: 0301-5629
- WATSON R J ET AL: "Classification of arterial plaque by spectral analysis of in vitro radio frequency intravascular ultrasound data" ULTRASOUND IN MEDICINE AND BIOLOGY, NEW YORK, NY, US, vol. 26, no. 1, 1 January 2000 (2000-01-01), pages 73-80, XP004295492 ISSN: 0301-5629
- KOMIYAMA NOBUYUKI ET AL: "Tissue characterization of atherosclerotic plaques by intravascular ultrasound radiofrequency signal analysis: An in vitro study of human coronary arteries" AMERICAN HEART JOURNAL, vol. 140, no. 4, October 2000 (2000-10), pages 565-574, XP002575181 ISSN: 0002-8703

## Description

### BACKGROUND OF THE INVENTION

### 1. Field of the Invention

The present invention relates to vascular tissue, or more particularly, to a system and method of using backscattered data and known parameters to characterize vascular tissue.

### 2. Description of Related Art

The present invention relates to the intra-vascular ultrasound (IVUS) analysis arts. It finds particular application to a system and method for quantitative component identification within a vascular object including characterization of tissue. It should be appreciated that while the present invention is described in terms of an ultrasonic device, or more particularly the use of IVUS data (or a transformation thereof) to characterize a vascular object, the present invention is not so limited. Thus, for example, using backscattered data (or a transformation thereof) to characterize any tissue type or composition is also contemplated.

Ultrasonic imaging of portions of a patient's body provides a useful tool in various areas of medical practice for determining the best type and course of treatment. Imaging of the coronary vessels of a patient by ultrasonic techniques can provide physicians with valuable information. For example, the image data may show the extent of a stenosis in a patient, reveal progression of disease, help determine whether procedures such as angioplasty or atherectomy are indicated or whether more invasive procedures may be warranted.

Document US6200268B1 discloses a method and system for characterizing plaque components within a vascular object. The vascular object is scanned with an ultrasonic device and backscatter signal data is collected. Histology images are prepared and digitized which correspond to the scanned vascular sections.

Another document "Assessing spectral algorithms to predict atherosclerotic plaque composition with normalized and raw intravascular ultrasound data" by Nair, A. et al., Ultrasound in Medicine and Biology, Vol. 27, No. 10, pp. 1319-1331, relates to the spectral analysis of backscattered intravascular data and the characterization of plaque.

In a typical ultrasound imaging system, an ultrasonic transducer is attached to the end of a catheter that is carefully maneuvered through a patient's body to a point of interest such as within a blood vessel. The transducer may be a single-element crystal or probe that is mechanically scanned or rotated back and forth to cover a sector over a selected angular range. Acoustic signals are then transmitted and echoes (or backscatter) from these acoustic signals are received. The backscatter data can be used to identify the type or density of a scanned tissue. As the probe is swept through the sector, many acoustic lines are processed building up a sector-shaped image of the patient. After the data is collected, an image of the blood vessel (i.e., an IVUS image) is reconstructed using well-known techniques. This image is then visually analyzed by a cardiologist to assess the vessel components and plaque content.

Typically, the ultrasonic image data is transferred to a VHS videotape, digitized and then analyzed. This process, however, loses image resolution since the videotape typically has a lower resolution than the originally collected backscatter data. Losing image resolution may result in an inaccurate evaluation of a vessel and its plaque content. Furthermore, certain image characteristics like brightness and contrast will be different for different patients or could vary for the same patient if the cardiologist varies the settings on the IVUS console. The images that are recorded on the videotapes are the same images viewed on the IVUS console screen and, thus, subject to the settings on the console. Since plaque (or tissue type) is identified by its appearance on the screen, errors may occur in the analysis if the screen settings have been modified. Another drawback is that certain information (e.g., tissue composition, etc.) cannot readily be discerned from an IVUS image (at least not to any degree of certainty). Thus, it would be advantageous to have a system and method of characterizing and/or imaging a vascular object that overcomes at least one of these drawbacks.

### SUMMARY OF THE INVENTION

The present invention provides a system according to claim 8 and a method according to claim 1 of using backscattered data and known parameters to characterize vascular tissue. Embodiments of the present invention operate in accordance with an ultrasonic device and a computing device comprising a characterization application and a database. Specifically, the ultrasonic device (e.g., intra-vascular ultrasound (IVUS) console and IVUS catheter) is used to acquire RF backscattered data (i.e., IVUS data) from a blood vessel. For example, a transducer may be attached to the end of a catheter and carefully maneuvered through a patient's body to a point of interest. The transducer is then pulsed to acquire echoes or backscattered signals (i.e., IVUS data) reflected from the tissue of the vascular object. The IVUS data is then transmitted to the computing device and used (either by the computing device or the IVUS console) to create an IVUS image.

According to the present invention, the characterization application is adapted to receive and store characterization data (e.g., tissue type data, etc.). Specifically, after the vascular object has been interrogated, the vascular object is cross-sectioned for histology. The cross-section is then prepared with a fixing and staining process that is well known in the art. The staining process allows a clinician to identify a tissue type(s). The identified tissue type (e.g., characterization data) is then provided to the characterization application and stored in the database.

Furthermore, according to the present invention, the characterization application is further adapted to create a histology image and identify at least one corresponding region on the IVUS image. In this embodiment, digitized data corresponding to the cross-sectioned vascular object is provided to the characterization application. The digitized data is then used to create a histology image. A region of interest (ROI) on the histology image is then identified by the operator. Preferably, the ROI corresponds to the characterization data, as previously provided. The characterization application is then adapted to identify a corresponding region on the IVUS image. To accurately match the ROI, however, it may be necessary to warp or morph the histology image to substantially fit the contour of the IVUS image. This warping removes histological preparation artifacts caused by cutting the tissue. Accordingly, according to the present invention, the characterization application is further adapted to morph the histology image by (i) identifying (or receiving identifying data from an operator on) at least one landmark common to both the histology image and the IVUS image, (ii) use a first algorithm (e.g., a morphometric algorithm) to substantially align the corresponding landmarks, and, preferably, (iii) use a second algorithm (e.g., a thin plate spline (TPS) deformation technique) to substantially align the non-landmark portions of the object.

According to the present invention, the characterization application is further adapted to determine and store parameters associated with the ROI portion of the IVUS image. In this embodiment, the characterization application is adapted to identify the IVUS data that corresponds to the ROI on the IVUS image. After the IVUS data has been identified, and in accordance with one embodiment of the present invention, the characterization application is adapted to identify at least one parameter of the IVUS data. According to the present invention, the characterization application is adapted to identify a plurality of parameters including integrated backscatter and at least one of maximum power, minimum power, frequency at maximum power, frequency at minimum power, y intercept, slope, and mid-band fit after frequency analysis has been performed (e.g., using fast Fourier transform, the Welch periodogram, autoregressive power spectrum (AR) analysis). The identified parameter is then stored in the database, where it is linked to the characterization data. This data (i.e., stored parameters and characterization data) can then be used to identify or characterize vascular tissue.

According to the present invention, the characterization application is adapted to receive IVUS data, determine parameters related thereto, and use the parameters stored in the database (i.e., histology data) to identify tissue type(s) or characterization(s) thereof. The characterization application is adapted to receive IVUS data from the IVUS console and identify at least one parameter associated therewith (either directly or indirectly). In other words, the parameters may be identified directly from the IVUS data or from a transformation thereof (e.g., after frequency analysis). The identified parameters are then compared to the parameters stored in the database (i.e., histology data). If a match (either exactly or substantially) is found, the related region is correlated to the tissue type (or characterization) stored in the database. In one embodiment of the present invention, the characterization application is further adapted to display a reconstructed image of the interrogated vascular object, where different tissue types are identified using different colors (e.g., discrete colors, gray-scales, etc.).

A more complete understanding of the system and method of using backscattered data and known parameters to characterize vascular tissue will be afforded to those skilled in the art, as well as a realization of additional advantages and objects thereof, by a consideration of the following detailed description of preferred embodiments. Reference will be made to the appended sheets of drawings which will first be described briefly.

### BRIEF DESCRIPTION OF THE DRAWINGS

Figure 1 illustrates a tissue-characterization system in accordance with one embodiment of the present invention, including an IVUS console, an IVUS catheter, a computing device and an input device.
Figure 2 illustrates an exemplary IVUS image.
Figure 3 illustrates a cross-section of an exemplary vascular object in-vivo and in-vitro.
Figure 4 illustrates an alternate embodiment of the computing device depicted in Figure 1.
Figure 5 illustrates an exemplary image of a characterized vascular object.
Figure 6 illustrates a method of characterizing a vascular object in accordance with one embodiment of the present invention.

### DETAILED DESCRIPTION OF THE PREFERRED EMBODIMENT

Preferred embodiments of the present invention operate in accordance with an ultrasonic device and a computing device comprising a characterization application and a database. In the detailed description that follows, like element numerals are used to describe like elements illustrated in one or more figures.

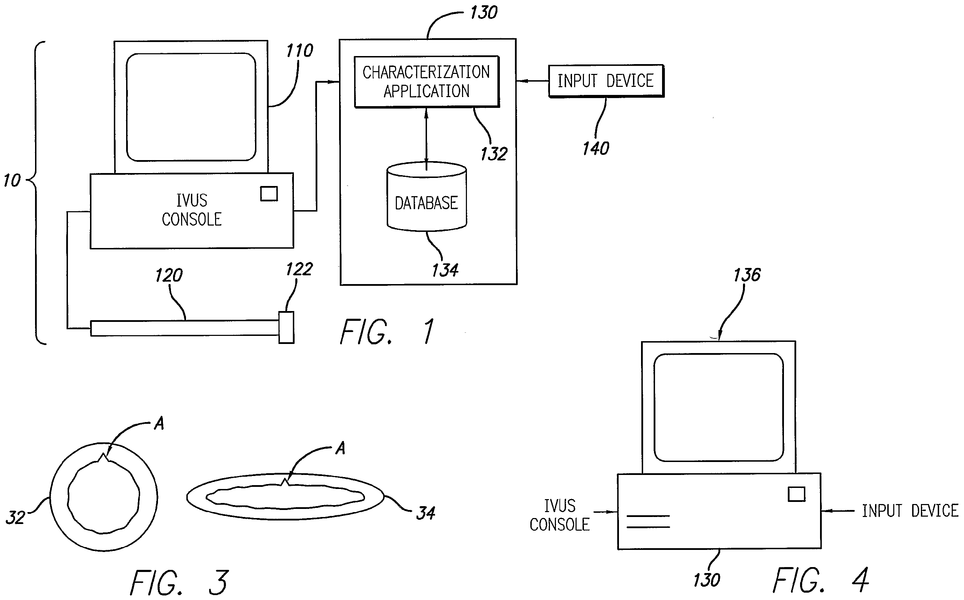

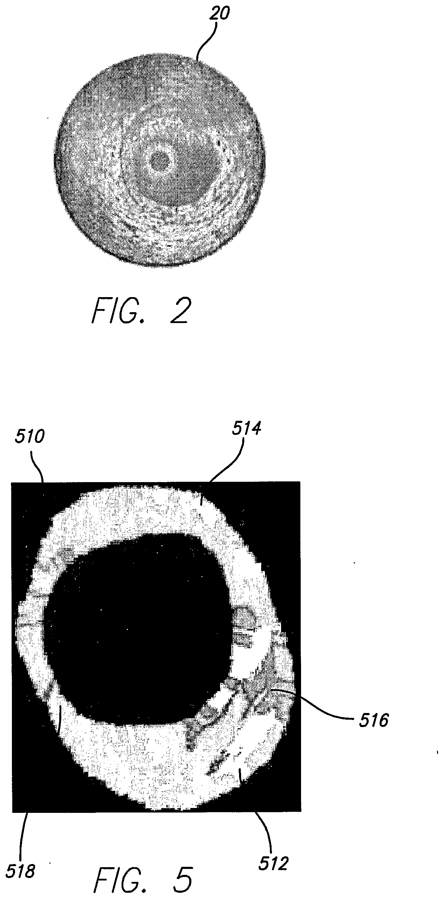

Figure 1 illustrates a tissue-characterization system 10 operating in accordance with the present invention. In this embodiment, an intra-vascular ultrasound (IVUS) console 110 is electrically connected to an IVUS catheter 120 and used to acquire RF backscattered data (i.e., IVUS data) from a blood vessel. Specifically, a transducer 122 is attached to the end of the catheter 120 and is carefully maneuvered through a patient's body to a point of interest. The transducer is then pulsed to acquire echoes or backscattered signals reflected from the tissue of the vascular object. Because different types and densities of tissue absorb and reflect the ultrasound pulse differently, the reflected data (i.e., IVUS data) can be used to image the vascular object. In other words, the IVUS data can be used (e.g., by the IVUS console 110) to create an IVUS image. An exemplary IVUS image 20 is provided in Figure 2, where the light and dark regions indicate different tissue types and/or densities. It should be appreciated that the IVUS console 110 depicted herein is not limited to any particular type of IVUS console, and includes all ultrasonic devices known to those skilled in the art (e.g., a C-VIS Clearview Imaging System, etc.). It should further be appreciated that the IVUS catheter 120 depicted herein is not limited to any particular type of catheter, and includes all ultrasonic catheters known to those skilled in the art. Thus, for example, a catheter having a single transducer (e.g., adapted for rotation) or an array of transducers (e.g., circumferentially positioned around the catheter) is also contemplated.

Referring back to Figure 1 , the tissue-characterization system 10 further includes a computing device 130 comprising a database 134 and a characterization application 132 electrically connected to the database 134 and adapted to receive IVUS data from the IVUS console 110. It should be appreciated that the database 134 depicted herein includes, but is not limited to, RAM, cache memory, flash memory, magnetic disks, optical disks, removable disks, SCSI disks, IDE hard drives, tape drives and all other types of data storage devices (and combinations thereof, such as RAID devices) generally known to those skilled in the art. It should further be appreciated that the characterization application 132, as depicted and discussed herein, may exist as a single application or as multiple applications, locally and/or remotely stored. It should also be appreciated that the number and location of the components depicted in Figure 1 are not intended to limit the present invention, and are merely provided to illustrate the environment in which the present invention may operate. Thus, for example, a computing device having a plurality of databases and/or a remotely located characterization application (either in part or in whole) is also contemplated.

In one embodiment of the present invention, the characterization application 132 is adapted to receive and store characterization data (e.g., tissue type, etc.). Specifically, after a vascular object has been interrogated (e.g., IVUS data has been collected), a histology correlation is prepared. In other words, the vascular object is dissected or cross-sectioned for histology. In one embodiment of the present invention, the cross-section is previously marked, for example with a suture, so that the histology can be corresponded to a portion of the IVUS image. The cross-section is then prepared with a fixing and staining process that is well known in the art. The staining process allows a clinician to identify a tissue type(s), or a chemical(s) found within (e.g., a chemical corresponding to a particular tissue type, etc.). It should be appreciated that the particular method used to identify or characterize the cross-sectional object is not a limitation of the present invention. Thus, all identification/characterization methods generally known to those skilled in the art are within the spirit and scope of the present invention.

The identified tissue type or characterization (i.e., characterization data) is then provided to the characterization application 132. In one embodiment, as shown in Figure 1, the characterization data is provided via an input device 140 electrically connected to the computing device 130. The characterization data is then stored in the database 134. It should be appreciated that the input device depicted herein includes, but is not limited to, a keyboard, a mouse, a scanner and all other data-gathering and/or data-entry devices generally known to those skilled in the art. It should further be appreciated that the term tissue type or characterization, as these terms are used herein, include, but are not limited to, fibrous tissues, fibro-lipidic tissues, calcified necrotic tissues, calcific tissues, collagen compositions, cholesterol, thrombus, compositional structures (e.g., the lumen, the vessel wall, the medial-adventitial boundary, etc.) and all other identifiable characteristics generally known to those skilled in the art.

In one embodiment of the present invention, the characterization application is adapted to create a histology image and to identify at least one corresponding region on an IVUS image. Specifically, digitized data is provided to the characterization application (e.g., via the input device 140), where the digitized data corresponds to the cross-sectioned vascular object. The digitized data is then used to create a histology image (i.e., a digital image or outline that substantially corresponds to the vascular object). A region of interest (ROI) on the histology image can then be identified by the operator. Preferably, the ROI is characterized by the characterization data, as previously provided, and may be the entire histology image or a portion thereof. The characterization application is then adapted to identify a corresponding region (e.g., x,y coordinates, etc.) on the IVUS image (i.e., the image created using the raw backscattered data, or the IVUS data).

To accurately match the ROI, however, the histology image may need to be warped to substantially fit the contour of the IVUS image. The warping removes histological preparation artifacts caused by cutting and/or fixing the tissue. For example, as shown in Figure 3, the shape of a in-vivo vascular object 32 is generally round. Once this object is cut, or cross-sectioned for histology (i.e., creating an in-vitro vascular object 34), the object may appear somewhat distorted, or flattened. Furthermore, the tissue may shrink (e.g., about 30%) when it is put through the fixation process. Thus, in order to identify a corresponding ROI on the IVUS image, the histology image may need to be warped or morphed, to return it to its original shape.

Accordingly, in one embodiment of the present invention, the characterization application is adapted to morph the histology image. Specifically, the characterization application is adapted to identify (or receive identifying data from an operator on) at least one landmark common to both the histology image and the IVUS image (see e.g., Figure 3, landmark A). The characterization application is then adapted to use (i) a first algorithm (e.g., a morphometric algorithm) to substantially align the corresponding landmarks and (ii) a second algorithm (e.g., a thin plate spline (TPS) deformation technique) to substantially align the non-landmark portions of the object. In other words, the second algorithm is used to shape the regions or boundaries between the landmarks. It should be appreciated that the landmarks discussed herein include, but are not limited to, side branch vessels, identifiable plaque or calcium deposits, and all other vascular tissue landmarks generally known to those skilled in the art.

In one embodiment of the present invention, the characterization application is further adapted to determine and store parameters associated with the ROI portion of the IVUS image. Specifically, the characterization application is adapted to identify the IVUS data (i.e., the raw backscatter data) that corresponds to the ROI identified on the IVUS image. In other words, the IVUS data that was originally used to create the ROI on the IVUS image is identified. In one embodiment of the present invention, the characterization application is further adapted to identify at least one parameter of the identified IVUS data. This parameter is then stored in the database, where it is linked to the characterization data. It should be appreciated, however, that each parameter may be associated with more than one tissue type or characterizations. For example, a first parameter may be common to multiple tissue types, thus requiring additional parameters to narrow the field.

In another embodiment of the present invention, signal analysis (i.e., frequency analysis, etc.) is performed on the identified IVUS data before the parameters are identified. In other words, the IVUS data is converted (or transformed) into the frequency domain (e.g., from the time domain) to identify the frequency spectrum of the ROI. This is because the frequency information obtained from the backscattered signal (or parameters associated therewith) can serve as a "signature" for each tissue type or characteristic. In one embodiment of the present invention, the frequency analysis is performed using a fast Fourier transform (FFT). In another embodiment of the present invention, the frequency analysis is performed using the Welch periodogram. In another embodiment of the present invention, the frequency analysis is performed using autoregressive power spectrum (AR) analysis. At least one parameter of the frequency-based signal is then identified and stored in the database, where it is linked to the characterization data.

In another embodiment of the present invention, both backscattered data (e.g., IVUS data) and its frequency spectrum are analyzed to classify the ROI portion of the IVUS image. Specifically, the frequency spectrum (or more particularly at least one parameter identified therefrom) is used to identify tissue type and the backscattered data is used to identify tissue location. This is because the backscatter data is in the time domain, and can thus be used to spatially identify certain frequencies (or parameters related thereto). For example, if a vascular wall comprises multiple tissue layers, corresponding backscattered data can be used to identify the location of these tissues and the related frequency spectrum can be used to identify tissue types. It should be appreciated that, while certain embodiments have been described in terms of frequency transformation, the present invention is not so limited. Thus, alternate transformations (e.g., wavelet transformation, etc.) are within the spirit and scope of the present invention. It should further be appreciated that the term parameter, as that term is used herein, includes, but is not limited to maximum power, minimum power, frequencies at maximum and/or minimum power, y intercepts (estimated or actual), slope, mid-band fit, integrated backscatter and all parameters generally known to (or discernable by) those skilled in the art. It should also be appreciated that the term "histology data," as that term is used herein, includes data stored in the database (e.g., characterization data, parameters, etc.).

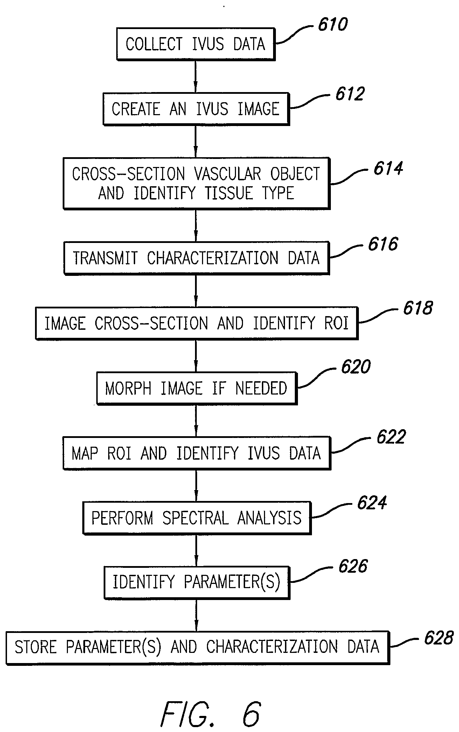

One method of populating the database is illustrated in Figure 6. Specifically, at step 610, IVUS data (i.e., RF backscatter data) is collected from a portion of a vascular object. This data is then used to create an IVUS image at step 612. At step 614, the interrogated portion of the vascular object is cross-sectioned and a tissue type (or a characterization thereof) is identified. This information (i.e., characterization data) is then transmitted to a computing device (or the equivalent thereof) at step 616. At step 618, an image of the cross-sectioned vascular object is created and a ROI is identified (e.g., by an operator). This image is then morphed, if needed, to substantially match the cross-section image to the IVUS image at step 620. This may include identifying at least one landmark and applying at least one algorithm (e.g., a morphometric algorithm, a TPS deformation technique, etc.). At step 622, the ROI is mapped to the IVUS image and associated IVUS data is identified. Spectral analysis is then performed on the associated IVUS data at step 624, and at least one parameter is identified at step 626. The at least one parameter and the characterization data is then stored at step 628. In one embodiment of the present invention, the at least one parameter is stored such that it is linked to the characterization data. It should be appreciated that the order in which these steps are presented is not intended to limit the present invention. Thus, for example, creating an IVUS image after the vascular object is cross-sectioned is also contemplated.

The above-described process is repeated for each tissue component desired to be identified and repeated for each component as many times as desired in order to obtain a more accurate range of signal properties. With the database populated, a tissue type or characteristic can be automatically and accurately identified if the acquired parameters substantially match parameters stored in the database.

Accordingly, in the present invention, the characterization application is adapted to receive IVUS data, determine parameters related thereto, and use the parameters stored in the database (i.e., histology data) to identify tissue type(s) or characterization(s) thereof. Specifically, with reference to Figure 1 , the characterization application 132 is adapted to receive IVUS data from the IVUS console 110. The characterization application 132 is then adapted to identify at least one parameter associated (either directly or indirectly) with the IVUS data. It should be appreciated that the IVUS data may either be received in real-time (e.g., while the patient is in the operating room) or after a period of delay (e.g., via CD-ROM, etc.). It should further be appreciated that the identified parameters should be related (generally) to the stored parameters. Thus, for example, an estimated Y intercept parameter should be identified if data related to a signal's estimated Y intercept is stored in the database 134 and linked to at least one tissue type. Moreover, if the stored parameters were acquired after frequency analysis was performed (i.e., are related to a frequency-based signal), then frequency analysis (preferably of the same type) should be performed on the IVUS data before parameters are identified. However, the IVUS data may be used to identify spatial information, as previously discussed.

The identified parameters are then compared to the parameters stored in the database (i.e., histology data). If a match (either exactly or substantially) is found, the related region is correlated to the tissue type (or characterization) stored in the database 134 (e.g., as linked to the matching parameters). It should be appreciated that a match may occur as long as the parameters fall within a range of properties for a particular tissue type found in the database.

In one embodiment, after each region is identified, the characterization application is further adapted to display a reconstructed image of the interrogated vascular object on a display. A computing device 130 including such a display 136 is illustrated in Figure 4. In one embodiment of the present invention, each tissue type (or characterization) is distinguished through the use of gray-scales or discrete colors. For example, Figure 5 illustrates an exemplary reconstructed vascular object 510, where different tissues (e.g., calcific tissues 512, fibrous tissues 514, calcified necrotic tissues 516 and fibro-lipidic tissues 518) are identified using different shades of gray. Such a system makes different tissue types or characterizations easily identifiable. Additional examples of characterized vascular objects are provided by U.S. Patent Number 6,200,268, which was issued on March 13, 2001. It should be appreciated that the reconstructed vascular object may further identify vascular borders. Systems and methods of identifying vascular borders are provided by U.S. Provisional Application Numbers, 60/406,184, 60/406,234, and 60/406,185, which were filed August 26, 2002, and by U.S. Patent Number 6,381,350, which issued April 30, 2002.

Having thus described embodiments of a system and method of using backscattered data and known parameters to characterize a vascular tissue, it should be apparent to those skilled in the art that certain advantages of the system have been achieved. It should also be appreciated that various modifications, adaptations, and alternative embodiments thereof are also contemplated within the scope of the invention, which is defined by the following claims.

## Claims

1. A method of characterizing vascular tissue, comprising;
receiving IVUS RF backscatter data collected previously (610) from a portion of a vascular object (616);
using (612) at least said RF backscatter data to construct a first image of said portion of said vascular object;
preparing (614) a histology of said portion of said vascular object with a fixing and staining process;
using (618) said histology to construct a second image of said portion of said vascular object;
characterizing at least a portion of said histology;
identifying a region of interest, ROI, of said second image, said ROI corresponding to said at least a portion of said histology;
identifying (620) at least one landmark (A) common to a corresponding contour of each of said first and second images and using said at least one landmark to identify a region of said first image that substantially corresponds to said ROI of said second image;
identifying (622) a portion of said RF backscatter data corresponding to said region of said first image;
performing a frequency transformation (624) on said portion of said RF backscatter data;
identifying (626) a plurality of parameters of said RF backscatter data including an integrated backscatter and at least one of: maximum power, minimum power, frequency at maximum power, frequency at minimum power, y intercept, slope, mid-band fit, wherein said performing a frequency transformation (624) is performed before said identifying said plurality of parameters of said RF backscatter data and such that said plurality of parameters are identified from the result of said frequency transformation; and
storing (628) said plurality of parameters and said characterization of said at least a portion of said histology;
wherein said step of identifying at least one landmark further comprises applying a morphometric algorithm to align the at least one landmark of said second image to substantially match the at least one landmark of said first image; and wherein the method further comprises:
receiving a second set of IVUS RF backscatter data collected previously from a second vascular object;
performing the frequency transformation on at least a portion of said second set of RF backscatter data to produce a third set of data;
identifying at least another parameter from a third set of data; and
using said at least another parameter, said plurality of parameters and said characterization of said at least a portion of said histology to characterize at least a portion of said second vascular object.

2. The method of Claim 1, wherein said step of performing a frequency transformation (624) further comprises using a fast Fourier transform (FFT).

3. The method of Claim 1, wherein said step of performing a frequency transformation (624) further comprises using the Welch periodogram

4. The method of Claim 1, wherein said step of performing a frequency transformation (624) further comprises using autoregressive power spectrum (AR) analysis.

5. The method of Claim 1, wherein said step of identifying at least one parameter (626) further comprises performing a wavelet transformation on said portion of said RF backscatter data before said at least one parameter is identified.

6. The method of Claim 1, wherein said step of identifying at least one landmark (620) further comprises aligning the non-landmark portions of said first and second images based on a thin plate algorithm.

7. The method of Claim 1, wherein said step of characterizing at least a portion of said histology further comprises identifying a tissue type, said tissue type being selected from a group consisting of fibrous tissues, fibro-lipidic tissues, calcified necrotic tissues, and calcific tissues.

8. A vascular-tissue-characterization system (10), comprising:
a computing device (130) comprising:
a database (134); and
a characterization application (132) electrically connected to said database (134) and adapted to:
receive intra-vascular ultrasound, IVUS, data corresponding to a portion of a vascular object and digitized data corresponding to a histology of said portion of said vascular object;
use at least said IVUS data and said digitized data to construct a first (20) and second image, respectively, of said portion of said vascular object;
receive characterization data corresponding to a region of interest (ROI) of said second image;
use at least one landmark (A) common to a corresponding contour of each of said first and second images to morph said second image to substantially match said first image and to identify said ROI on said first image;
identify a portion of said IVUS data corresponding to said ROI on said first image;
perform a spectral analysis on said IVUS data;
identify a plurality of parameters related to said portion of said IVUS data including an integrated backscatter and at least one of: maximum power, minimum power, frequency at maximum power, frequency at minimum power, y intercept, slope, mid-band fit, wherein the spectral analysis on said IVUS data is performed before said plurality of parameters are identified and such that said plurality of parameters are identified from the result of said spectral analysis; and
store said plurality of parameters and said characterization data in said database;
said characterization application (132) being further adapted to use a morphometric algorithm to align the at least one landmark of said second image to substantially match the at least one landmark of said first image; and wherein said characterization application (132) is further adapted to:
receive a second set of RF backscatter data collected previously from a second vascular object;
perform the spectral analysis on at least a portion of said second set of RF backscatter data to produce a third set of data;
identify at least another parameter from said third set of data; and
use said at least another parameter, said plurality of parameters and said characterization of said at least a portion of said histology to characterize at least a portion of said second vascular object.

9. The vascular-tissue-characterization system of Claim 8, wherein said characterization application (132) is further adapted to perform the Welch periodogram.

10. The vascular-tissue-characterization system of Claim 8, wherein said characterization application (132) is further adapted to analyze said IVUS data to identify at least one location corresponding to said at least one parameter.

11. The vascular-tissue-characterization system of Claim 8, wherein said characterization application is further adapted to perform a fast Fourier transform (FFT).

12. The vascular-tissue-characterization system of Claim 11, wherein said characterization application is further adapted to perform autoregressive power spectrum (AR) analysis.

13. The vascular-tissue-characterization system of Claim 8, wherein said characterization data comprises a tissue type, said tissue type being selected from a group consisting of fibrous tissues, fibro-lipidic tissues, calcified necrotic tissues, and calcific tissues.

14. The vascular-tissue-characterization system of Claim 8, further comprising an input device (140) electrically connected to said computing device, said characterization data being provided by said input device (140).

15. The vascular-tissue-characterization system of Claim 8, further comprising an IVUS console (110) adapted to:
acquire said IVUS data from said vascular object; and
provide said IVUS data to said computing device.

16. The vascular-tissue-characterization system of Claim 15, further comprising an IVUS catheter (120) having at least one transducer, said IVUS catheter being electrically connected to said IVUS console and adapted to acquire said IVUS data from said vascular object.

17. The vascular-tissue-characterization system of Claim 8, wherein said characterization application (132) is further adapted to align the non-landmark portions of said first and second images based on a thin plate algorithm.

## Patentansprüche

1. Verfahren zur Charakterisierung von Gefäßgewebe, umfassend:
Empfangen von IVUS-HF-Rückstreudaten, die zuvor (610) aus einem Abschnitt eines vaskulären Objekts (616) erfasst wurden;
Verwenden (612) von mindestens den genannten HF-Rückstreudaten, um ein erstes Bild des genannten Abschnitts des genannten vaskulären Objekts zu konstruieren;
Präparieren (614) einer Histologie des genannten Abschnitts des genannten vaskulären Objekts mit einem Fixier- und Einfärbungsprozess;
Verwenden (618) der genannten Histologie, um ein zweites Bild des genannten Abschnitts des genannten vaskulären Objekts zu konstruieren;
Charakterisieren von mindestens einem Abschnitt der genannten Histologie;
Identifizieren einer interessierenden Region (engl. region of interest, ROI) des genannten zweiten Bilds, wobei die genannte ROI dem genannten mindestens einen Abschnitt der genannten Histologie entspricht;
Identifizieren (620) von mindestens einem Orientierungspunkt (A), der einer entsprechenden Kontur von jedem von dem genannten ersten und zweiten Bild gemeinsam ist, und Verwenden des genannten mindestens einen Orientierungspunkts, um eine Region des genannten ersten Bilds zu identifizieren, die im Wesentlichen der genannten ROI des genannten zweiten Bilds entspricht;
Identifizieren (622) eines Teils der genannten HF-Rückstreudaten, der der genannten Region des genannten ersten Bilds entspricht;
Durchführen einer Frequenztransformation (624) für den genannten Teil der genannten HF-Rückstreudaten;
Identifizieren (626) einer Vielzahl von Parametern der genannten HF-Rückstreudaten einschließlich einer integrierten Rückstreuung und mindestens einem von: maximaler Leistung, minimaler Leistung, Frequenz bei maximaler Leistung, Frequenz bei minimaler Leistung, y-Achsenabschnitt, Steigung, Bandmittenpassung, wobei das genannten Durchführen einer Frequenztransformation (624) vor dem genannten Identifizieren der genannten Vielzahl von Parametern der genannten HF-Rückstreudaten durchgeführt wird und derartig, dass die genannte Vielzahl von Parametern anhand des Ergebnisses der genannten Frequenztransformation identifiziert wird; und
Speichern (628) der genannten Vielzahl von Parametern und der genannten Charakterisierung des genannten mindestens einen Abschnitts der genannten Histologie;
wobei der genannte Schritt des Identifizierens von mindestens einem Orientierungspunkt ferner das Anwenden eines morphometrischen Algorithmus umfasst, um den mindestens einen Orientierungspunkt des genannten zweiten Bilds so auszurichten, dass er im Wesentlichen mit dem mindestens einen Orientierungspunkt des genannten ersten Bilds übereinstimmt; und wobei das Verfahren ferner Folgendes umfasst:
Empfangen eines zweiten Satzes von IVUS-HF-Rückstreudaten, die zuvor aus einem zweiten vaskulären Objekt erfasst wurden;
Durchführen der Frequenztransformation für mindestens einen Teil des genannten zweiten Satzes von HF-Rückstreudaten, um einen dritten Datensatz zu erzeugen;
Identifizieren von mindestens einem weiteren Parameter aus einem dritten Datensatz; und
Verwenden des genannten mindestens einen weiteren Parameters, der genannten Vielzahl von Parametern und der genannten Charakterisierung des genannten mindestens einen Abschnitts der genannten Histologie, um mindestens einen Abschnitt der genannten zweiten vaskulären Objekts zu charakterisieren.

2. Verfahren nach Anspruch 1, wobei der genannte Schritt des Durchführens einer Frequenztransformation (624) ferner das Verwenden einer Fast-FourierTransformation (FFT) umfasst.

3. Verfahren nach Anspruch 1, wobei der genannte Schritt des Durchführens einer Frequenztransformation (624) ferner das Verwenden des Welch-Periodogramms umfasst.

4. Verfahren nach Anspruch 1, wobei der genannte Schritt des Durchführens einer Frequenztransformation (624) ferner das Verwenden von autoregressiver Leistungsspektrum (AR)-Analyse umfasst.

5. Verfahren nach Anspruch 1, wobei der genannte Schritt des Identifizierens von mindestens einem Parameter (626) ferner das Durchführen einer Wavelet-Transformation für den genannten Teil der genannten HF-Rückstreudaten vor dem Identifizieren des genannten mindestens einen Parameters umfasst.

6. Verfahren nach Anspruch 1, wobei der genannte Schritt des Identifizierens von mindestens einem Orientierungspunkt (620) ferner das Ausrichten der Nicht-Orientierungspunkt-Abschnitte des genannten ersten und zweiten Bilds basierend auf einem Thin-Plate-Algorithmus umfasst.

7. Verfahren nach Anspruch 1, wobei der genannte Schritt des Charakterisierens von mindestens einem Abschnitt der genannten Histologie ferner das Identifizieren eines Gewebetyps umfasst, wobei der genannte Gewebetyp ausgewählt wird aus einer Gruppe bestehend aus fibrösem Gewebe, Fibrolipid-Gewebe, kalzifiziertem nekrotischem Gewebe und kalzifiziertem Gewebe.

8. System (10) zur Charakterisierung von vaskulärem Gewebe, umfassend:
eine Computervorrichtung (130), umfassend:
eine Datenbank (134); und
eine Charakterisierungsanwendung (132), die mit der genannten Datenbank (134) elektronisch verbunden ist und ausgelegt ist zum:
Empfangen von intravaskulären Ultraschalldaten (IVUS-Daten), die einem Abschnitt eines vaskulären Objekts entsprechen, und digitalisierten Daten, die einer Histologie des genannten Abschnitts des genannten vaskulären Objekts entsprechen;
Verwenden von mindestens den genannten IVUS-Daten und den genannten digitalisierten Daten, um ein erstes (20) Bild bzw. zweites Bild des genannten Abschnitts des genannten vaskulären Objekts zu konstruieren;
Empfangen von Charakterisierungsdaten, die einer interessierenden Region (ROI) des genannten zweiten Bilds entsprechen;
Verwenden eines ersten Orientierungspunkts (A), der einer entsprechenden Kontur von jedem von dem genannten ersten und zweiten Bild gemeinsam ist, um das genannte zweite Bild so zu verformen, dass es im Wesentlichen mit genannten ersten Bild übereinstimmt, und um die genannte ROI in dem genannten ersten Bild zu identifizieren;
Identifizieren eines Teils der genannten IVUS-Daten, der der genannten ROI im genannten ersten Bild entspricht;
Durchführen einer Spektralanalyse für die genannten IVUS-Daten;
Identifizieren einer Vielzahl von Parametern, die sich auf den genannten Teil der genannten IVUS-Daten beziehen, einschließlich einer integrierten Rückstreuung und mindestens einem von: maximaler Leistung, minimaler Leistung, Frequenz bei maximaler Leistung, Frequenz bei minimaler Leistung, y-Achsenabschnitt, Steigung, Bandmittenpassung, wobei die Spektralanalyse der genannten IVUS-Daten durchgeführt wird, bevor die genannte Vielzahl von Parametern identifiziert wird, und derartig, dass die genannte Vielzahl von Parametern anhand des Ergebnisses der genannten Spektralanalyse identifiziert wird; und
Speichern der genannten Vielzahl von Parametern und der genannten Charakterisierungsdaten in der genannten Datenbank;
wobei die genannte Charakterisierungsanwendung (132) ferner dafür ausgelegt ist, einen morphometrischen Algorithmus zu verwenden, um den mindestens einen Orientierungspunkt des genannten zweiten Bilds so auszurichten, dass er im Wesentlichen mit dem mindestens einen Orientierungspunkt des genannten ersten Bilds übereinstimmt; und wobei die genannte Charakterisierungsanwendung (132) ferner ausgelegt ist zum:
Empfangen eines zweiten Satzes von HF-Rückstreudaten, die zuvor aus einem zweiten vaskulären Objekt erfasst wurden;
Durchführen der Spektralanalyse für mindestens einen Teil des genannten zweiten Satzes von HF-Rückstreudaten, um einen dritten Datensatz zu erzeugen;
Identifizieren von mindestens einem weiteren Parameter aus dem genannten dritten Datensatz; und
Verwenden des genannten mindestens einen weiteren Parameters, der genannten Vielzahl von Parametern und der genannten Charakterisierung des genannten mindestens einen Abschnitts der genannten Histologie, um mindestens einen Abschnitt der genannten zweiten vaskulären Objekts zu charakterisieren.

9. System zur Charakterisierung von vaskulärem Gewebe nach Anspruch 8, wobei die genannte Charakterisierungsanwendung (132) ferner dafür ausgelegt ist, das Welch-Periodogramm durchzuführen.

10. System zur Charakterisierung von vaskulärem Gewebe nach Anspruch 8, wobei die genannte Charakterisierungsanwendung (132) ferner dafür ausgelegt, die genannten IVUS-Daten zu analysieren, um mindestens einen Ort zu identifizieren, der dem genannten einen Parameter entspricht.

11. System zur Charakterisierung von vaskulärem Gewebe nach Anspruch 8, wobei die genannte Charakterisierungsanwendung ferner dafür ausgelegt, eine Fast-Fourier-Transformation (FFT) durchzuführen.

12. System zur Charakterisierung von vaskulärem Gewebe nach Anspruch 11, wobei die genannte Charakterisierungsanwendung ferner dafür ausgelegt ist, autoregressive Leistungsspektrum (AR)-Analyse durchzuführen.

13. System zur Charakterisierung von vaskulärem Gewebe nach Anspruch 8, wobei die genannten Charakterisierungsdaten einen Gewebetyp umfassen, wobei der genannte Gewebetyp ausgewählt wird aus einer Gruppe bestehend aus fibrösem Gewebe, Fibrolipid-Gewebe, kalzifiziertem nekrotischem Gewebe und kalzifiziertem Gewebe.

14. System zur Charakterisierung von vaskulärem Gewebe nach Anspruch 8, ferner umfassend eine Eingabevorrichtung (140), die mit der genannten Computervorrichtung elektrisch verbunden ist, wobei die genannten Charakterisierungsdaten durch die genannte Eingabevorrichtung (140) bereitgestellt werden.

15. System zur Charakterisierung von vaskulärem Gewebe nach Anspruch 8, ferner umfassend eine IVUS-Konsole (110), die ausgelegt ist zum:
Erfassen der genannten IVUS-Daten aus dem genannten vaskulären Objekt; und
Bereitstellen der genannten IVUS-Daten für die genannte Computervorrichtung.

16. System zur Charakterisierung von vaskulärem Gewebe nach Anspruch 15, ferner umfassend ein IVUS-Katheter (120) mit mindestens einem Wandler, wobei der genannte IVUS-Katheter mit der genannten IVUS-Konsole elektrisch verbunden ist und dafür ausgelegt ist, die genannten IVUS-Daten aus dem genannten vaskulären Objekt zu erfassen.

17. System zur Charakterisierung von vaskulärem Gewebe nach Anspruch 8, wobei die genannte Charakterisierungsanwendung (132) ferner dafür ausgelegt ist, die Nicht-Orientierungspunkt-Abschnitt des genannten ersten und zweiten Bilds basierend auf einem Thin-Plate-Algorithmus auszurichten.

## Revendications

1. Procédé de caractérisation d'un tissu vasculaire, comprenant de :
recevoir des données de rétrodiffusion RF IVUS collectées au préalable (610) à partir d'une partie d'un objet vasculaire (616) ;
utiliser (612) au moins lesdites données de rétrodiffusion RF pour construire une première image de ladite partie dudit objet vasculaire ;
préparer (614) une histologie de ladite partie dudit objet vasculaire à l'aide d'un processus de fixation et de coloration ;
utiliser (618) ladite histologie pour construire une seconde image de ladite partie dudit objet vasculaire ;
caractériser au moins une partie de ladite histologie ;
identifier une région d'intérêt, ROI, de ladite seconde image, ladite ROI correspondant à ladite au moins une partie de ladite histologie ;
identifier (620) au moins un point de repère (A) commun à un contour correspondant de chacune desdites première et seconde images et utiliser ledit au moins un point de repère pour identifier une région de ladite première image qui correspond sensiblement à ladite ROI de ladite seconde image ;
identifier (622) une partie desdites données de rétrodiffusion RF correspondant à ladite région de ladite première image ;
effectuer une transformation de fréquence (624) sur ladite partie desdites données de rétrodiffusion RF ;
identifier (626) une pluralité de paramètres desdites données de rétrodiffusion RF, y compris une rétrodiffusion intégrée et au moins l'un parmi : une puissance maximale, une puissance minimale, une fréquence à puissance maximale, une fréquence à puissance minimale, une ordonnée à l'origine, une pente, un ajustement à mi-bande, dans lequel ladite étape consistant à effectuer une transformation de fréquence (624) est effectuée avant ladite identification de ladite pluralité de paramètres desdites données de rétrodiffusion RF et de telle sorte que ladite pluralité de paramètres est identifiée d'après le résultat de ladite transformation de fréquence ; et
stocker (628) ladite pluralité de paramètres et ladite caractérisation de ladite au moins une partie de ladite histologie ;
dans lequel ladite étape d'identification d'au moins un point de repère comprend en outre l'application d'un algorithme morphométrique pour aligner le au moins un point de repère de ladite seconde image afin de le faire sensiblement correspondre au au moins un point de repère de ladite première image ; et dans lequel le procédé comprend en outre de :
recevoir un deuxième ensemble de données de rétrodiffusion RF IVUS collectées au préalable à partir d'un second objet vasculaire ;
effectuer la transformation de fréquence sur au moins une partie dudit deuxième ensemble de données de rétrodiffusion RF afin de produire un troisième ensemble de données ;
identifier au moins un autre paramètre à partir d'un troisième ensemble de données ; et
utiliser ledit au moins un autre paramètre, ladite pluralité de paramètres et ladite caractérisation de ladite au moins une partie de ladite histologie pour caractériser au moins une partie dudit second objet vasculaire.

2. Procédé selon la revendication 1, dans lequel ladite étape consistant à effectuer une transformation de fréquence (624) comprend en outre l'utilisation d'une transformée de Fourier rapide (FFT).

3. Procédé selon la revendication 1, dans lequel ladite étape consistant à effectuer une transformation de fréquence (624) comprend en outre l'utilisation du périodogramme de Welch.

4. Procédé selon la revendication 1, dans lequel ladite étape consistant à effectuer une transformation de fréquence (624) comprend en outre l'utilisation d'une analyse de spectre de puissance autorégressive (AR).

5. Procédé selon la revendication 1, dans lequel ladite étape d'identification d'au moins un paramètre (626) comprend en outre d'effectuer une transformation en ondelettes sur ladite partie desdites données de rétrodiffusion RF avant que ledit au moins un paramètre soit identifié.

6. Procédé selon la revendication 1, dans lequel ladite étape d'identification d'au moins un point de repère (620) comprend en outre d'aligner les parties sans point de repère desdites première et seconde images sur la base d'un algorithme de plaques minces.

7. Procédé selon la revendication 1, dans lequel ladite étape de caractérisation d'au moins une partie de ladite histologie comprend en outre l'identification d'un type de tissu, ledit type de tissu étant sélectionné parmi un groupe constitué de tissus fibreux, de tissus fibro-lipidiques, de tissus nécrotiques calcifiés, et de tissus calcifiants.

8. Système de caractérisation de tissu vasculaire (10), comprenant :
un dispositif informatique (130) comprenant :
une base de données (134) ; et
une application de caractérisation (132) connectée électriquement à ladite base de données (134) et adaptée pour :
recevoir des données d'échographie intra-vasculaire, IVUS, correspondant à une partie d'un objet vasculaire et des données numérisées correspondant à une histologie de ladite partie dudit objet vasculaire ;
utiliser au moins lesdites données IVUS et lesdites données numérisées pour construire, respectivement, une première image (20) et une seconde image de ladite partie dudit objet vasculaire ;
recevoir des données de caractérisation correspondant à une région d'intérêt (ROI) de ladite seconde image ;
utiliser au moins un point de repère (A) commun à un contour correspondant de chacune desdites première et seconde images pour transformer ladite seconde image pour qu'elle corresponde sensiblement à ladite première image et pour identifier ladite ROI sur ladite première image ;
identifier une partie desdites données IVUS correspondant à ladite ROI sur ladite première image ;
effectuer une analyse spectrale sur lesdites données IVUS ;
identifier une pluralité de paramètres liés à ladite partie desdites données IVUS, y compris une rétrodiffusion intégrée et au moins l'un parmi : une puissance maximale, une puissance minimale, une fréquence à puissance maximale, une fréquence à puissance minimale, une ordonnée à l'origine, une pente, un ajustement à mi-bande, dans lequel l'analyse spectrale sur lesdites données IVUS est effectuée avant l'identification de ladite pluralité de paramètres et de sorte que ladite pluralité de paramètres est identifiée d'après le résultat de ladite analyse spectrale ; et
stocker ladite pluralité de paramètres et lesdites données de caractérisation dans ladite base de données ;
ladite application de caractérisation (132) étant en outre adaptée pour utiliser un algorithme morphométrique afin d'aligner le au moins un point de repère de ladite seconde image pour le faire sensiblement correspondre au au moins un point de repère de ladite première image ; et dans lequel ladite application de caractérisation (132) est en outre adaptée pour :
recevoir un deuxième ensemble de données de rétrodiffusion RF collectées au préalable à partir d'un second objet vasculaire ;
effectuer l'analyse spectrale sur au moins une partie dudit deuxième ensemble de données de rétrodiffusion RF afin de produire un troisième ensemble de données ;
identifier au moins un autre paramètre à partir dudit troisième ensemble de données ; et
utiliser ledit au moins un autre paramètre, ladite pluralité de paramètres et ladite caractérisation de ladite au moins une partie de ladite histologie pour caractériser au moins une partie dudit second objet vasculaire.

9. Système de caractérisation de tissu vasculaire selon la revendication 8, dans lequel ladite application de caractérisation (132) est en outre adaptée pour effectuer le périodogramme de Welch.

10. Système de caractérisation de tissu vasculaire selon la revendication 8, dans lequel ladite application de caractérisation (132) est en outre adaptée pour analyser lesdites données IVUS afin d'identifier au moins un emplacement correspondant audit au moins un paramètre.

11. Système de caractérisation de tissu vasculaire selon la revendication 8, dans lequel ladite application de caractérisation est en outre adaptée pour effectuer une transformation de Fourier rapide (FFT).

12. Système de caractérisation de tissu vasculaire selon la revendication 11, dans lequel ladite application de caractérisation est en outre adaptée pour effectuer une analyse de spectre de puissance autorégressive (AR).

13. Système de caractérisation de tissu vasculaire selon la revendication 8, dans lequel lesdites données de caractérisation comprennent un type de tissu, ledit type de tissu étant sélectionné parmi un groupe constitué de tissus fibreux, de tissus fibro-lipidiques, de tissus nécrotiques calcifiés, et de tissus calcifiants.

14. Système de caractérisation de tissu vasculaire selon la revendication 8, comprenant en outre un dispositif d'entrée (140) connecté électriquement audit dispositif informatique, lesdites données de caractérisation étant fournies par ledit dispositif d'entrée (140).

15. Système de caractérisation de tissu vasculaire selon la revendication 8, comprenant en outre une console IVUS (110) adaptée pour :
acquérir lesdites données IVUS à partir dudit objet vasculaire ; et
fournir lesdites données IVUS audit dispositif informatique.

16. Système de caractérisation de tissu vasculaire selon la revendication 15, comprenant en outre un cathéter IVUS (120) ayant au moins un transducteur, ledit cathéter IVUS étant connecté électriquement à ladite console IVUS et adapté pour acquérir lesdites données IVUS à partir dudit objet vasculaire.

17. Système de caractérisation de tissu vasculaire selon la revendication 8, dans lequel ladite application de caractérisation (132) est en outre adaptée pour aligner les parties sans point de repère desdites première et seconde images sur la base d'un algorithme de plaques minces.
